# EUROPEAN PATENT SPECIFICATION

(11) **EP 4 026 585 B1**
(45) Date of publication and mention of the grant of the patent: **18.06.2025**
(21) Application number: 22150543.1
(22) Date of filing: 07.01.2022
(51) Int. Cl.: A61N 1/362, A61N 1/365, A61N 1/37, A61N 1/39

(54) **SYSTEMS FOR PACING ASSISTANCE AND AUTOMATION**
SYSTEME ZUR SCHRITTUNTERSTÜTZUNG UND AUTOMATISIERUNG
SYSTÈMES D'ASSISTANCE ET D'AUTOMATISATION DE STIMULATION

(30) Priority: 08.01.2021 US 202163199563 P
(43) Date of publication of application: 13.07.2022
(73) Proprietor: Stryker Corporation, Portage, MI 49002-9711 (US)
(72) Inventor: WALKER, Robert G., Redmond, 98052 (US); KRUSOR, Blaine, Redmond, 98052 (US); LINVILLE, David J., Redmond, 98052 (US); PIRAINO, Daniel W., Redmond, 98052 (US); SULLIVAN, Joseph L., Redmond, 98052 (US); TAYLOR, Tyson G., Redmond, 98052 (US)
(74) Representative: V.O.

(56) References cited:
- US-A1- 2006 173 499
- US-A1- 2010 010 551
- US-A1- 2012 035 676
- US-A1- 2016 175 598
- US-A1- 2019 160 278

## Description

### BACKGROUND

Pacing is a technique by which electrical stimulation is delivered to a heart of a patient to control and/or modify a rate at which the heart beats. In some cases, pacing may be administered to a patient experiencing bradycardia, or slow heart beats, via electrodes applied to an external surface of the patient, where the electrodes are connected to an external pacing device. In some examples, the external pacing device also functions as a defibrillator. An objective of pacing is to achieve capture of the heart of the patient. Capture is achieved when the electrical stimulation delivered during pacing causes depolarization of the ventricles of the heart (electrical capture) and/or contraction of the myocardium of the heart (mechanical capture). In conventional systems, a healthcare provider may operate the pacing device to place the electrodes on the skin of the patient, administer pacing, and determine whether capture has been achieved. Administering pacing and determining whether capture has been achieved presents challenges for healthcare providers, especially in stressful situations where a patient's life may be at risk.

US 2019/160278 A1 shows a transcutaneous pacing and defibrillation system measuring ECG signals and heart sound signals to control stimulation. US 2016/175598 A1 shows a wearable pacing device externally pacing the heart and measuring ECG and heart sound signals. US 2010/010551 A1 shows a transcutaneous cardiac stimulation system that delivers pacing pulses to a patient through body-surface electrodes according to a cardioprotective pacing protocol taking into account ECG signals.

### SUMMARY

The present invention provides an external pacing according to claim 1. The dependent claims define embodiments of the present invention.

Methods disclosed hereinafter are not claimed but useful for understanding the invention. The scope of the present invention is defined by the appended claims. determining that the likelihood is greater than or equal to the threshold likelihood, controlling the discharge circuit to provide a second electrical stimulation at the current level by the discharge circuit to the electrodes. The processor can e.g. be thus configured by performing instructions, e.g. stored on one or more computer readable media. In use, the first electrical stimulation can be provided to an external surface of the patient by the discharge circuit via the electrodes. In use, the second electrical stimulation can be provided to the patient via the electrodes.

Optionally, the first data associated with the first biological parameter comprises: a T-wave or QRS complex identified in an electrocardiogram (ECG); a plethysmography signal identified in an oxygen saturation (SpO2) measurement; a non-invasive blood pressure (NIBP) signal identified in an NIBP measurement; a regional cerebral oxygen saturation (rSO2) signal identified in an rSO2 measurement; an invasive blood pressure signal identified in an invasive blood pressure measurement; an end-tidal CO2 (ETCO2) signal identified in an ETCO2 measurement; and a capnography signal identified in a respiratory measurement.

Optionally, the second data associated with the second biological parameter comprises a different one of the T-wave or the QRS complex, the plethysmography signal, the NIBP signal, the rSO2 signal, the invasive blood pressure signal, the ETCO2 signal, or the capnography signal than the first biological parameter.

Optionally, the system further comprises a user interface. The user interface can be configured for: displaying a first representation of the first biological parameter; displaying a second representation of the second biological parameter with the first representation of the first biological parameter; and displaying an indicator overlaying the first representation and the second representation at the first time at which the first electrical stimulation was provided.

Optionally, the processor is further configured for: determining a first delay associated with receiving the first data from the first biological sensor; and determining a second delay associated with receiving the second data from the second biological sensor, wherein a location of the indicator relative to the first representation and the second representation is based on the first delay and the second delay.

Optionally, determining the likelihood comprises: inputting the first data associated with the first biological parameter and the second data associated with the second biological parameter into a machine-learned model trained to identify heart capture; and receiving, from the machine-learned model, the likelihood that the first electrical stimulation has caused the capture of the heart of the patient. The machine-learned model can comprise a logistic regression model.

Optionally, the capture corresponds to electrical capture or mechanical capture of the heart of the patient.

Optionally, the processor is further configured for: receiving, at a third time prior to the first time at which the electrical stimulation was provided, third data associated with the first biological parameter; and determining a difference between the third data associated with the first biological parameter and first data associated with the biological parameter received at the second time, wherein determining the likelihood is further based on the difference. Determining the likelihood can comprise: inputting the first data associated with the first biological parameter and the difference into a machine-learned model trained to identify heart capture; and receiving, from the machine-learned model, the likelihood that the first electrical stimulation has caused the capture of the heart of the patient.

Optionally, the first data associated with the first biological parameter comprises an indication of the electrical stimulation, the processor further being configured for: determining a magnitude of a response of the patient to the electrical stimulation from the first data; removing the magnitude of the response from the first data, wherein determining the likelihood is further based on removing the magnitude of the response from the first data.

The present invention can be used in an exemplary, non-claimed method, the method comprising: providing, via an external pacing device at a first time, a first electrical stimulation at a first current level to an external surface of a patient; receiving, from a first biological sensor at a second time after the first time, first data associated with a first biological parameter of the patient; receiving, from a second biological sensor at the second time, second data associated with a second biological parameter of the patient, the second biological parameter different from the first biological parameter; determining, based on the first data and the second data received at the second time, a likelihood that the first electrical stimulation has caused capture of a heart of the patient; comparing the likelihood to a threshold likelihood; and responsive to determining that the likelihood is less than the threshold likelihood, providing a second electrical stimulation at a second current level via the external pacing device, the second current level being different than the first current level.

### BRIEF DESCRIPTION OF THE DRAWINGS

FIG. 1 illustrates an example pacing device configured to provide pacing assistance and automation according to the techniques described herein.
FIG. 2 illustrates an example pacing device displaying an example user interface indicating a time at which an electrical stimulation was administered across multiple biological parameters, according to the techniques described herein.
FIG. 3 illustrates an example pacing device displaying an example user interface including a notification to display an additional biological parameter, according to the techniques described herein.
FIG. 4 is an example method for providing electrical stimulation based on a likelihood of capture determined from multiple biological parameters, according to the techniques described herein.
FIG. 5 is an example method for providing electrical stimulation based on sensor data collected at different times, according to the techniques described herein.
FIG. 6 is an example method for displaying a representation of a biological parameter based on a determination that pacing is being administered, according to the techniques described herein.
FIG. 7 illustrates an example of an external defibrillator configured to perform various functions described herein.

### DETAILED DESCRIPTION

Various implementations described herein relate to providing pacing assistance and automation. For example, a pacing device, such as an external defibrillator, provides electrical stimulations to an external surface of a patient based on a determination as to whether capture has been achieved. As used herein, an "electrical stimulation" is an electrical signal output by the pacing device at a current between, for example, 0 and 200 milliamperes (mA), delivered at a frequency such as 60 paces per minute (PPM). In some examples, the pacing device provides electrical stimulation at an initial current of 60mA, and pacing capture is achieved at or near 70mA.

The pacing device determines whether capture has been achieved using one or multiple types of sensor data (e.g., electrocardiogram (ECG), oxygen saturation (SpO₂), non-invasive blood pressure (NIBP), invasive blood pressure, end-tidal CO₂ (ETCO₂), regional cerebral oxygen saturation (rSO₂), and/or ventilation), and/or historical sensor data, for instance. These techniques effect a particular treatment (e.g., pacing using an external pacing system) for a medical condition (e.g., bradycardia). In some examples, the pacing device provides notifications to assist with pacing to a healthcare provider that is administering pacing to a patient. Examples of notifications include an indication that a representation corresponding to a biological parameter of a patient may assist with pacing, that the patient has an internal pacemaker, and so forth. These techniques improve the functioning of the pacing device by displaying an application summary of un-launched representations that would assist the healthcare provider with pacing, where conventional systems would require the healthcare provider to navigate through multiple menus to locate the un-launched representations while the patient's health rapidly deteriorates.

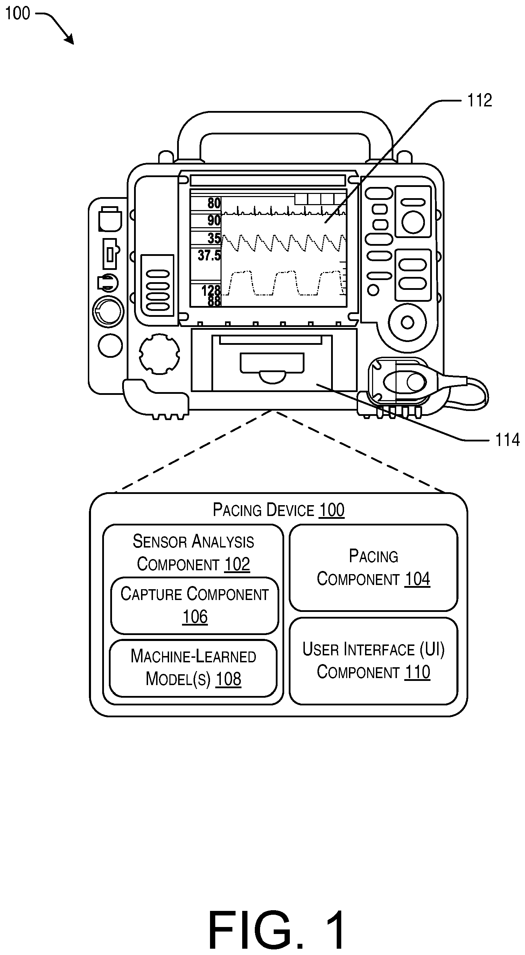

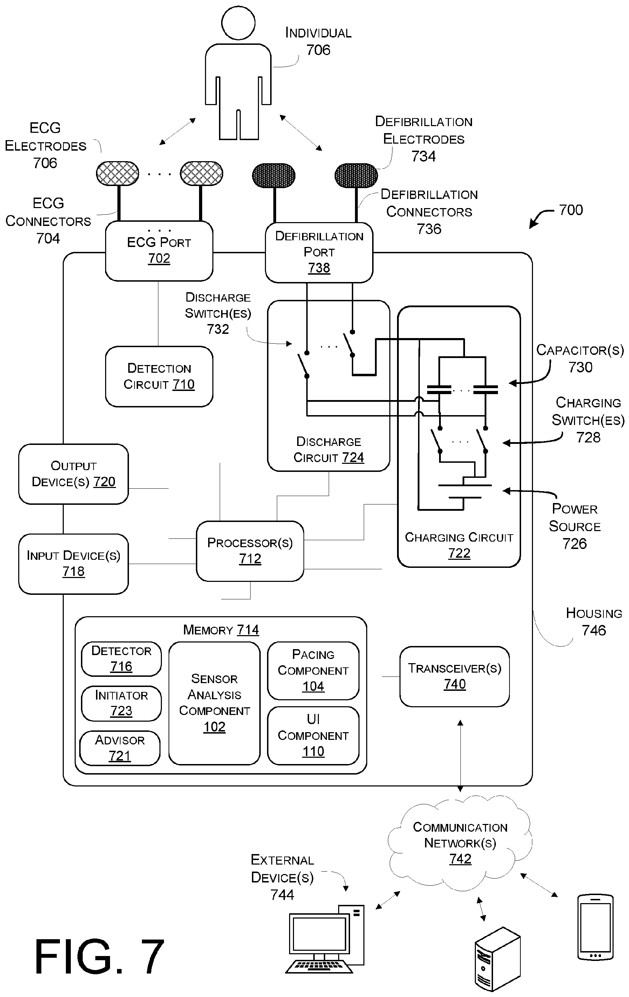

FIG. 1 illustrates an example pacing device 100 configured to provide pacing assistance and automation according to the techniques described herein. In some examples, the pacing device 100 is also an external defibrillator having functionality similar to the external defibrillator 700 described in relation to FIG. 7. The pacing device 100 is configured to administer pacing in the form of electrical stimulation at intervals to electrodes attached to a patient via electrode leads (not pictured), such as to treat bradycardia. The pacing device 100 is also configured to receive data associated with biological parameters, such as ECG, SpO₂, NIBP, invasive blood pressure, ETCO₂, rSO₂, ventilation, and so on from various biological sensors that collect the data associated with the respective biological parameters. As described in more detail below, the pacing device 100 includes various components to perform the functionality described herein. The components described herein in relation to the pacing device 100 correspond to software (digital) circuits, and/or analog circuits configured to perform the described functionality.

The pacing device 100 includes a sensor analysis component 102 that is configured to analyze data received from various types of sensors, such as biological sensors. For example, the sensor analysis component 102 receives data associated with biological parameters configured to sense the biological parameters, as described above. In examples, the sensor analysis component 102 analyzes the data associated with the biological parameters to detect characteristics of the biological parameters. For instance, in response to receiving ECG data, the sensor analysis component 102 detects the presence (or absence) of a T-wave, which is a shape of an ECG waveform that is indicative of repolarization of the ventricles of a heart of a patient. Alternatively or additionally, in response to receiving ECG data, the sensor analysis component 102 detects the presence (or absence) of a QRS complex, which is a representation of electrical impulse as it spreads through the ventricles of the heart and indicates ventricular depolarization. In some cases, the sensor analysis component 102 foregoes detection of specific complexes, waves, or other indicators in ECG data, and instead compares how similar the ECG data is between electrical stimulations during pacing.

In response to receiving SpO₂ data, the sensor analysis component 102 detects the presence (or absence) of a plethysmography signal (e.g., a waveform), which is a representation of the absorption of light by tissue, including blood, between an emitter and a sensor. The sensor analysis component 102 derives an oxygen saturation for the plethysmography signal from one or more light absorption curves. In response to receiving NIBP data, the sensor analysis component 102 detects the presence (or absence) of a non-invasive blood pressure signal (e.g., a waveform). In some cases, the NIBP data includes a signal corresponding to how full a bladder of a blood pressure cuff is (e.g., based on an amount of pressure within the blood pressure cuff). In response to receiving invasive blood pressure data, the sensor analysis component 102 detects the presence (or absence) of an invasive blood pressure signal (e.g., a waveform). In response to receiving ventilation data, the sensor analysis component 102 detects the presence (or absence) of a capnography signal (e.g., a waveform). In some instances, the sensor analysis component 102 receives sensor data from non-biological sensors as well, such as from a therapy board of the pacing device 100 that provides data indicating locations of impulses generated by the pacing device 100, thereby providing the pacing device 100 with information about when to expect an artifact from electrical stimulations. Other examples of characteristics that the sensor analysis component 102 may detect from sensor data are also considered.

The pacing device 100 also includes a pacing component 104 configured to cause a discharge circuit (not pictured) of the pacing device 100 to deliver an electrical stimulation to electrodes (also referred to as "ECG electrodes") via electrode leads (also referred to as "ECG leads") attached to the pacing device 100. The pacing component 104 controls a current and/or a voltage of the electrical stimulation delivered to the electrodes. In some examples, the pacing component 104 delivers pulses of electrical stimulation at regular (or irregular) intervals to the electrodes in response to a pacing setting of the pacing device 100 being activated. The pacing component 104 may control any of the voltage, the current, or the interval at which the electrical stimulations are delivered based on data received by the sensor analysis component 102, and/or characteristics detected by the sensor analysis component 102 from the data. This may include the pacing component 104 automatically modifying (e.g., without user intervention) any of the voltage, the current, or the interval at which the electrical stimulations are delivered based on data received by the sensor analysis component 102, and/or characteristics detected by the sensor analysis component 102 from the data.

In some examples, the sensor analysis component 102 includes a capture component 106 configured to determine, based on the sensor data, a likelihood that capture has been achieved during pacing. As used herein, "capture" may refer to electrical capture and/or mechanical capture. The capture component 106 may determine the likelihood that capture has been achieved based on data associated with one biological parameter received by the sensor component 102 from a biological sensor, or multiple biological parameters received by the sensor component 102 from different ones of the biological sensors. By using multiple different biological parameters to determine a likelihood that capture has been achieved, the capture component 106 improves accuracy of a capture determination over systems that may rely on a single biological parameter. However, examples are also considered in which the capture component 106 utilizes a single biological parameter (e.g., ECG) to determine a likelihood that capture has been achieved, such as if a second biological sensor is malfunctioning or unavailable.

Alternatively or additionally, the capture component 106 may determine the likelihood that capture has been achieved based on historical data associated with one or multiple biological parameters. In conventional systems, a healthcare provider who is administering pacing is provided with a limited duration (e.g., 10 seconds, 30 seconds, etc.) of sensor data on a monitor associated with a pacing device which is updated continuously as time progresses, and/or may generate a printout of historical sensor data. The healthcare provider in such conventional systems must then interpret the sensor data as it passes along on the display and/or interpret the separate printout, if used, along with continuing to administer pacing to the patient. In some cases, when pacing is being administered, it can be helpful to determine the likelihood that capture has been achieved by analyzing biological parameters prior to a time at which pacing administration began. However, these conventional systems cause difficulty and frustration for healthcare providers during pacing administration, by either not displaying a long enough duration of the biological parameter to view prior to and/or during administration of pacing, or by manually searching through a printout of the historical sensor data to locate a relevant time span. Accordingly, the capture component 106 compares historical sensor data (e.g., prior to pacing administration, at a previously administered current level, etc.) to recent sensor data (e.g., within the last 30 seconds, a time period during which a present current level has been administered, etc.) to determine a likelihood that capture has been achieved.

In some cases, the capture component 106 may employ a rules-based system to determine the likelihood that capture has been achieved. Such a rules-based system includes thresholds associated with different biological parameters (e.g., in a database or look-up table). The capture component 106 receives data associated with biological parameters before, during, and/or after pacing is administered to a patient, and compares values included in the data to the thresholds to determine a likelihood that capture has been achieved.

In an illustrative example, the sensor analysis component 102 may receive data associated with an ECG and sensor data associated with SpO₂. The sensor component 102 provides the data (and/or characteristics determined from the sensor data, such as signals that may include waveforms) to the capture component 106. The capture component 106 compares values in the ECG data, such as changes in values of the ECG data between electrical stimulations. Alternatively or additionally, the capture component 106 determines a presence (or absence) of a T-wave (or a QRS-like fluctuation) in a fixed time period (e.g., 300 milliseconds), and/or compares an amplitude of a T-wave to a threshold amplitude of a T-wave associated with successful capture (e.g., 4mm, 5mm, etc.).

Additionally, the capture component 106 compares values in the SpO₂ data or plethismograph, such as changes in the plethismographic waveform data between electrical stimulations, while accounting for delay for an electrical stimulation to induce cardiac electrical activity that will lead to a mechanical pulse generated by the heart , and for the mechanical pulse to travel to a sensor location of the SpO₂ sensor (e.g., at a fingertip). In the illustrative example, the capture component 106 determines that capture has been achieved if the changes in values of the ECG data between electrical stimulations received from the sensor analysis component 102 is greater than a threshold difference (e.g., greater than 10% difference), and if the changes in values of the SpO₂ data between electrical stimulations is greater than a threshold difference (e.g., greater than 10% difference). If the changes in values of the ECG data between electrical stimulations received from the sensor analysis component 102 is less than the threshold difference, and the changes in values of the SpO₂ data between electrical stimulations is less than the threshold difference, the capture component 106 determines that capture has not been achieved.

Alternatively or in addition to the rules-based system just described, the capture component 106 may employ machine-learned model(s) 108 to determine the likelihood that capture has been achieved. The machine-learned models 108 also utilize thresholds associated with different biological and/or device generated parameters, but in some cases the parameters of the machine-learned models 108 are modified to improve accuracy of capture likelihood determinations. For instance, the capture component 106 receives data associated with biological parameters and inputs at least a portion of this data into the machine-learned models 108.

In some examples, the machine-learned models 108 comprise one or more supervised models (e.g., classification, regression, similarity, or other type of model), unsupervised models (e.g., clustering, neural network, or other type of model), and/or semi-supervised models configured to determine a likelihood that capture has been achieved. For instance, the machine-learned model 108 may be a logistic regression model that outputs a likelihood that capture has been achieved based on the sensor data associated with the biological parameter(s). An example likelihood may include a value between 0 and 1, where the value corresponds to the likelihood that capture has been achieved and, when combined with a value representing the likelihood that capture has not been achieved, is equal to 1. In some cases, the sensor analysis component 102 converts the value between 0 and 1 representing the likelihood that capture has been achieved to a percentage value (e.g., 70% likelihood that capture has been achieved).

As discussed above, the sensor analysis component 102 may also leverage historical data of the patient to determine a likelihood of capture. For instance, the sensor analysis component 102 may receive data associated with biological parameter(s) of the patient prior to administration of pacing, at a previous electrical current level of electrical stimulation, and so forth. The pacing component 104 may also, in some examples, provide historical data including voltage, current, timing, and/or interval information to the sensor analysis component 102 regarding electrical stimulations that have been administered to the patient. The sensor analysis component 102 inputs the historical data into the machine-learned model 108 to provide information to the machine-learned model for determining whether capture has been achieved. The sensor analysis component 102 may continue to input data associated with one or multiple biological parameters over time, such that the machine-learned model 108 can provide a likelihood as to whether capture has been achieved. Additionally, the sensor analysis component 102 also inputs electrical stimulation data into the machine-learned model 108 over time, such that the machine-learned model 108 can refine the likelihood that capture has been achieved. In some examples, if the pacing device 100 did not administer pacing (e.g., in the form of electrical stimulation to electrodes attached to a patient via electrode leads), yet the sensor analysis component 102 detects the presence of a QRS complex, the sensor analysis component 102 may receive (or select) historical data associated with the heartbeat (e.g., intrinsic heartbeats) of the patient, and the sensor analysis component 102 may determine the likelihood of capture based on such historical data.

In examples, the pacing component 104 controls an electrical current level of an electrical stimulation administered to a patient via the electrodes based on a likelihood that capture has been achieved. The pacing component 104 compares the likelihood that capture has been achieved to at least one threshold likelihood of capture to determine whether an electrical current level of the electrical stimulation being delivered to the patient should change or remain the same for the upcoming electrical stimulation pulse. In an illustrative example, the pacing component 104 may utilize a single threshold (e.g., 70%, 80%, 90%, etc.) likelihood that capture has been achieved. In this example, the pacing component 104 compares a likelihood of capture of a particular patient received from the sensor analysis component 102 to the single threshold, and if the likelihood of capture is greater than or equal to the threshold, the pacing component 104 keeps the electrical current level the same for an upcoming electrical stimulation. However, if the likelihood of capture is less than the threshold, the pacing component 104 increases the electrical current level for an upcoming electrical stimulation (e.g., by 10 milliamps). In this way, the pacing device 100 controls the electrical current level of electrical stimulation administered to the patient based on a likelihood that capture has been achieved.

In another illustrative example, the pacing component 104 may utilize multiple threshold likelihoods that capture has been achieved. For instance, the pacing component 104 may employ a first threshold associated with capture being achieved, a second threshold associated with capture improving, and a third threshold associated with capture failure. In this example, the pacing component 104 compares a likelihood of capture of a particular patient received from the sensor analysis component 102 to the different thresholds. If the likelihood of capture is greater than or equal to the first threshold, the pacing component 104 keeps the electrical current level the same for an upcoming electrical stimulation. If the likelihood of capture is less than the first threshold and greater than or equal to the second threshold, the pacing component 104 increases the electrical current level for an upcoming electrical stimulation by a first amount (e.g., 10 milliamps). If the likelihood of capture is less than the second threshold, the pacing component 104 increases the electrical current level for an upcoming electrical stimulation by a second amount that is greater than the first amount (e.g., 15 milliamps). In this way, the pacing device 100 controls the electrical current level of electrical stimulation administered to the patient based on a likelihood that capture has been achieved, and how far the patient is from capture.

Although the examples above generally relate to modifying a current level of electrical stimulation based on a likelihood of capture, examples are also considered in which the pacing component 104 modifies a timing of electrical stimulations delivered to the patient as well. For instance, the pacing component 104 compares a likelihood of capture of a particular patient received from the sensor analysis component 102 to a threshold, as described above. If the likelihood of capture is greater than or equal to the threshold, the pacing component 104 keeps an interval between electrical stimulations the same for an upcoming electrical stimulation. However, if the likelihood of capture is less than the threshold, the pacing component 104 modifies the interval between electrical stimulations for an upcoming electrical stimulation (e.g., by decreasing a time between electrical stimulations by 50 milliseconds, by 500 milliseconds, etc.).

The sensor analysis component 102 continues to receive data from biological sensors associated with one or multiple biological parameters following administration of a particular electrical stimulation during pacing. In some cases, the sensor analysis component 102 determines a difference between a value associated with a biological parameter (e.g., an ECG wave amplitude) prior to and after the electrical stimulation was administered. After administration of an electrical stimulation during pacing, the capture component 106 again evaluates a likelihood that capture has been achieved using the previously administered electrical current level, using the techniques described herein. In examples, the capture component 106 uses the difference between values associated with the biological parameter prior to and after the electrical stimulation to determine the likelihood of capture. For instance, the capture component 106 inputs the difference into the machine-learned model 108 to use in determining the likelihood that the electrical stimulation caused capture. The pacing component 104 administers another electrical stimulation based on the likelihood that capture has been achieved by the previous electrical stimulation, which may include maintaining the same electrical current level, increasing the electrical current level, or decreasing electrical current level.

In addition to using data prior to and after a particular pacing pulse, the sensor analysis component 102 may analyze whether pacing is improving a condition of the patient over a longer time period. In some examples, the sensor analysis component 102 determines a difference between sensor data (e.g., an ECG signal) generated by the various sensors that detect biological conditions as described above after an electrical stimulation was administered and historical sensor data collected prior to administration of electrical stimulation during pacing. If the difference between the sensor data after the electrical stimulation was administered and historical sensor data collected prior to administration of electrical stimulation during pacing is greater than or equal to a threshold difference (e.g., 50%, 20%, 10% difference, etc.), the capture component 106 determines that the electrical stimulation has likely caused capture. Similar to the discussion above, the capture component 106 may input the difference into the machine-learned model 108, and receive the likelihood from the machine-learned model based on the difference. The pacing component 104 then causes the same electrical stimulation to be administered to the patient, as the electrical stimulation is likely causing capture and improving the heartbeat of the patient. If the difference between the sensor data after the electrical stimulation was administered and historical sensor data collected prior to administration of electrical stimulation during pacing is less than the threshold difference, the capture component 106 determines that the electrical stimulation has likely failed to cause capture. The pacing component 104 then causes the electrical stimulation being administered to the patient to increase, as the electrical stimulation is likely insufficient to improve the magnitude of the patient's heartbeat.

In some cases, data associated with different biological parameters is collected at different sampling rates, and different biological parameters have periodic cycles that have different time periods. For instance, a patient may have a heart rate of 70 beats per minute where a heartrate sensor samples the heart rate 120 times per minute, and a ventilation rate of 15 breaths per minute where a ventilation sensor samples the ventilation rate 20 times per minute. When using data associated with multiple different biological parameters, the sensor analysis component 102 determines the likelihood that capture has been achieved using data received at such different time periods. In an illustrative example, the sensor analysis component 102 may receive data associated with a first T-wave of an ECG at a first time, and data associated with a second T-wave of an ECG at a second time that is 0.8 seconds after the first time. The sensor analysis component 102 may then receive data associated with capnography signal that includes a waveform of a breath at a third time that is 0.5 seconds after the second time. The capture component 106 determines a likelihood that capture has been achieved using the data received at different times, such as by determining a first likelihood using the data associated with the first and second T-waves, then refining the likelihood once the data associated with the capnography signal is received. In some examples, the capture component 106 inputs the data associated with the different biological parameters received at different times into the machine-learned model 108, which returns the likelihood that capture has been achieved during pacing.

In some examples, the capture component 106 accounts for electrical stimulations administered to the patient that appear in the data received from the biological sensors. For instance, in ECG measurements, electrical stimulations often cause a "blank" in the data generated by the ECG sensor when the electrical stimulation is administered, because the electrical stimulation is significantly more intense than the electrical output of the heart and thus more powerful than the ECG sensor can record. Therefore, the capture component 106 determines a magnitude of a response of the patient to the electrical stimulation as indicated in the data received from the biological sensor. Continuing with the ECG example, the magnitude of the response corresponds to a difference in the electrical stimulation that was administered and the amplitude of the ECG signal at the time that the electrical stimulation was administered. The capture component 106 removes the magnitude of the response from the data associated with the biological parameter prior to determining the likelihood that capture was achieved as a result of the electrical stimulation. In this way, impact of the electrical stimulation itself on sensor data is reduced, and the accuracy of the sensor data associated with the biological parameter is increased.

The user interface (UI) component 110 may display a variety of information in association with biological parameters and/or pacing administration on a user interface 112 of the pacing device 100, and/or a remote computing device (not pictured). For example, the UI component 110 displays values associated with blood pressure, pulse rate, ETCO₂, ventilation rate, and the like, along with signals that may include waveforms representative of ECG, blood pressure, ETCO₂, and so on over time. In examples, the UI component 110 displays an indication of the likelihood that capture has been achieved during pacing, based on data associated with multiple biological parameters and/or a comparison of recent sensor data to historical sensor data as described above. The indication of the likelihood may assist a healthcare provider in cases where the healthcare provider is administering pacing and controlling the current level manually by combining information associated with multiple biological parameters into a single indicator of whether capture has been achieved, and if not, how to increase the likelihood of achieving capture. The UI component 110 can display the indication of the likelihood that capture has been achieved during pacing as a percentage likelihood (e.g., 25% likelihood, 50% likelihood, 75% likelihood, etc.), as a binary indication of capture (e.g., "Capture Achieved" or "Capture Not Achieved"), as a gauge indicator, as a bar indicator, and so forth. In some cases, the UI component also displays a recommendation of how to modify a current of the electrical stimulation being delivered to the patient during pacing, and/or an interval between electrical stimulations, based on a determination by the capture component 106 that modifying the electrical stimulation will increase the likelihood of capture.

In some examples, the UI component 110 allows different configurations of representations of various biological parameters on the user interface 112. For instance, the UI component 110 is configured to display more representations of biological parameters than those illustrated in the user interface 112, fewer representations of biological parameters than those illustrated in the user interface 112, representations of biological parameters in different locations on the user interface 112 than illustrated, and so on. In an illustrative example, a healthcare provider may choose to display a single representation of a biological parameter in the user interface 112, such as the ECG waveform. As noted above, however, having information associated with multiple biological parameters displayed simultaneously during pacing may assist a healthcare provider with determining whether capture has been achieved, and/or whether to modify the electrical stimulations that are delivered during pacing.

Accordingly, in examples, the UI component 110 determines that pacing is being administered and that fewer and/or different representations of biological parameters are currently being displayed than a predefined set of biological parameter representations that are helpful to identify capture. For instance, the predefined set of biological parameter representations may include ECG, SpO₂, and ETCO₂, although other examples are also considered (and are not limited to three representations). In response, the UI component 110 causes additional representation(s) of biological parameter(s) to be displayed on the user interface 112 that were previously absent from the user interface 112. Referencing the illustrative example above, the UI component 110 may cause an SpO₂ (plethysmography) waveform and an ETCO₂ trendline or capnography waveform to be displayed on the user interface 112 after pacing administration begins. In some examples, the UI component 110 displays a notification that additional representation(s) are absent from being displayed in the user interface 112 after determining that pacing is being administered, and prior to displaying the additional representation(s). The notification may include a selectable control that, when selected, causes the additional representation(s) to be displayed in the user interface 112. The notification may also include an additional selectable control that, when selected, causes the additional representation(s) to not be displayed in the user interface 112, which may be desirable to healthcare providers who have representations of the other biological parameters displayed elsewhere. In some cases, the UI component 110 outputs visual feedback, audio feedback, and/or haptic feedback with the notification, to draw the attention of the healthcare provider that additional information is available to be displayed on the user interface 112 during pacing.

The UI component 110 may include other information in the notification (or in a different notification) as well. For example, in response to the sensor analysis component 102 determining that the patient has an internal pacemaker, the UI component 110 outputs a notification on the user interface 112 indicating that the patient has an internal pacemaker. Such a notification may also include recommendations on whether and/or how to administer pacing differently to a patient with an internal pacemaker than to a patient without an internal pacemaker.

In some examples, the UI component 110 controls which representations of biological parameters to display based on a quality of the sensor data received from the various sensors. For instance, if a sensor is placed incorrectly or is malfunctioning, the data may inaccurately represent the biological parameter and thus cause confusion on behalf of the healthcare provider during pacing. Therefore, the UI component 110 applies quality thresholds to the sensor data, such as whether a sensor is providing data, whether the sensor data is greater than or less than a threshold for a particular biological parameter (e.g., ECG data with an average frequency greater than 150 Hz), whether there is an artifact detected in the sensor data (e.g., excessive electromagnetic, muscle, or motion artifact), and the like. If the UI component 110 determines that the sensor data does not meet the quality threshold, the UI component withholds the notification to display a representation associated with the sensor data of the particular biological parameter. Alternatively or additionally, the UI component 110 removes the representation associated with the sensor data of the particular biological parameter if previously displayed on the user interface 112 to avoid confusion by the healthcare provider, and/or displays a notification that the representation associated with the sensor data of the particular biological parameter is poor quality, such as with a recommendation to remove the particular biological parameter from the display. If the UI component 110 determines that the sensor data does meet the quality threshold, the UI component provides the notification to display a representation associated with the sensor data of the particular biological parameter, or in some cases displays the representation of the biological parameter automatically without first displaying a notification.

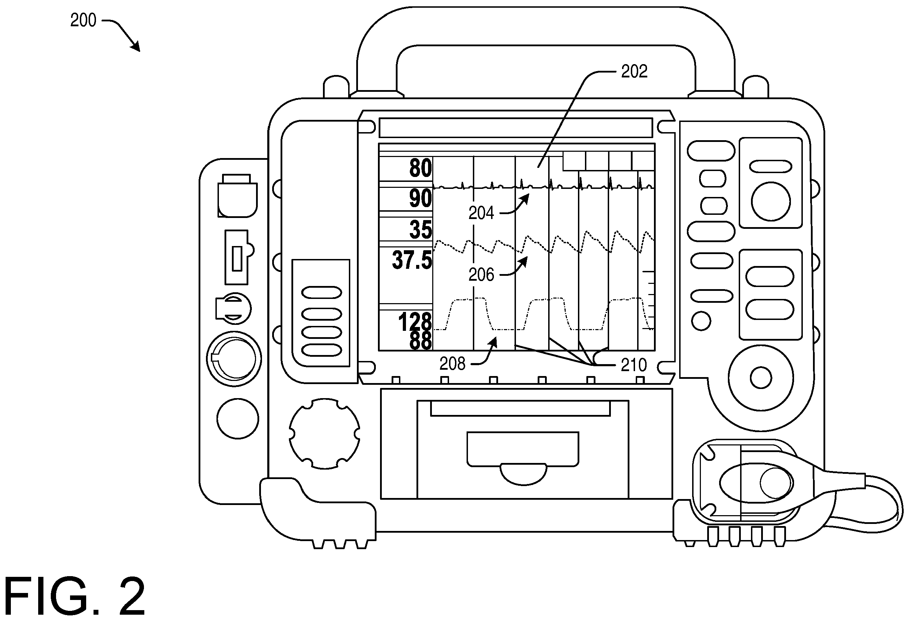

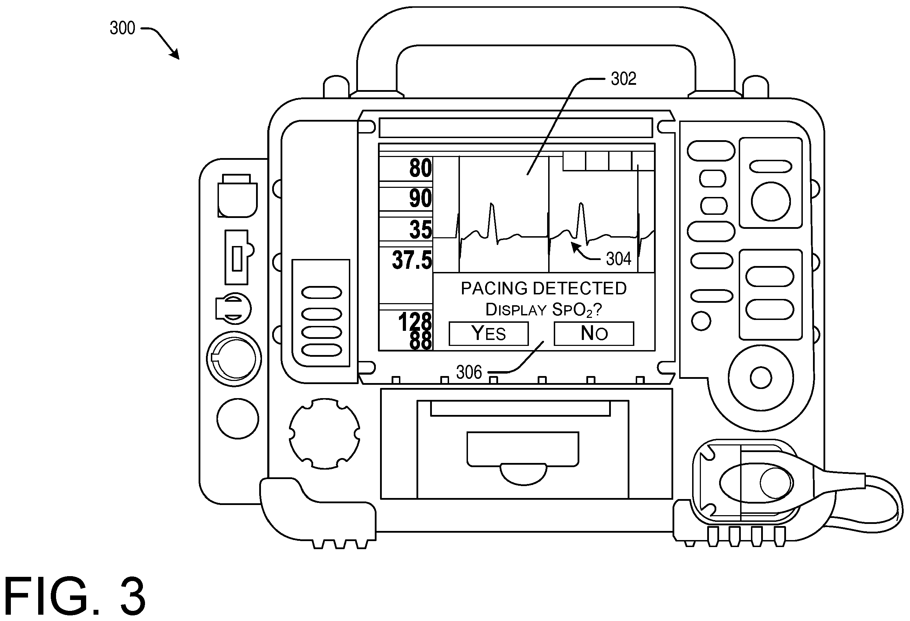

In many cases, the pacing device 100 includes a printing device 114 that enables a healthcare provider to print historical data associated with biological parameter(s). Because the user interface 112 displays a limited time period of the various representations of biological parameters and continues to update over time, printing the historical data provides the healthcare provider with more information about the biological parameters, such as a status of the biological parameters prior to administration of pacing, a status of the biological parameters in response to previous electrical stimulations during pacing, and so forth. Accordingly, in response to receiving a user input to print historical data associated with the biological parameter(s), the UI component 110 causes the printing device 114 to print a representation of the historical data. For example, the printing device 114 prints a representation of ECG data prior to pacing initiation, and prints a representation of the ECG data during pacing, and/or when one or more parameters (e.g., a current) of an electrical stimulation is adjusted. In some examples, the UI component 110 includes additional information in the printed representation, such as indications of intervals between heartbeats of the patient (and/or breaths of the patient), indications of when a pulse associated with pacing occurred, and so forth. In this way, the UI component 110 provides additional information to the healthcare provider that is easily interpreted to assist the healthcare provider in determining whether a condition of the patient is improving during pacing. Additional details regarding functionality of the UI component 110 are discussed in relation to FIG. 2 and FIG. 3.

For example, FIG. 2 illustrates a pacing device 200 displaying a user interface 202 indicating a time at which an electrical stimulation was administered across multiple biological parameters, according to the techniques described herein. The pacing device 200 may correspond to the pacing device 100 described in relation to FIG. 1. Accordingly, reference is made in the description of the pacing device 200 to various components described in relation to FIG. 1.

The user interface 202 includes various example representations of different biological parameters. A representation 204 illustrates ECG sensor data, and includes multiple T-waves corresponding to heartbeats of a patient. A representation 206 illustrates SpO₂ sensor data, and includes multiple plethysmography waves also corresponding to heartbeats of the patient. A representation 208 illustrates CO₂ sensor data, and includes capnography waveforms associated with changes in cardiac output of the patient. The user interface 202 also includes indicators 210 that overlay the representation 204, the representation 206, and the representation 208, where the indicators 210 correspond to times at which electrical stimulation was provided to the patient during pacing. In some examples, the UI component 110 displays the representation 204, the representation 206, the representation 208 (collectively, "representations 204, 206, and 208"), and the indicators 210 by accounting for delays in receiving the respective signals from the biological sensors providing the ECG sensor data, the SpO₂ sensor data, and the CO₂ sensor data. In an illustrative example, the UI component 110 may determine that the ECG sensor data is received at a 50 millisecond delay, and the SpO₂ sensor data is received at a 200 millisecond delay, such as based on responses detected by the sensor analysis component 102 to administration of the electrical stimulation pulses during pacing. Accordingly, the UI component 110 displays the indicators 210 at locations relative to the representations 204, 206, and 208 such that the indicators 210 appear at a same time in each of the representations 204, 206, and 208. This may include the UI component delaying display of a representation of biological parameter(s), such as by delaying output of the ECG sensor data by 150 milliseconds to account for the delay in the illustrative example above.

FIG. 3 illustrates a pacing device 300 displaying a user interface 302 including a notification to display a representation of an additional biological parameter, according to the techniques described herein. The pacing device 300 may correspond to the pacing device 100 described in relation to FIG. 1. Accordingly, reference is made in the description of the pacing device 300 to various components described in relation to FIG. 1.

The user interface 302 includes a representation 304 of a single biological parameter, in this case an ECG waveform. As discussed above, the UI component 110 detects that pacing is being administered, and determines which biological parameter(s) are present in the user interface 302. The UI component 110 determines that additional biological parameters are absent from the user interface 302 that are included in a predefined set of biological parameter representations that are helpful to identify capture. In response, the UI component 110 displays a notification 306 in the user interface 302 that pacing has been initiated by the pacing device 100, and includes a suggestion of an additional biological parameter (e.g., SpO₂) that can be displayed in the user interface 302 to assist the healthcare provider with identifying capture. The notification 306 includes a "Yes" selectable control that, when selected, causes a representation of the additional biological parameter(s) to be displayed in the user interface 302 with the representation 304. The notification 306 also includes a "No" selectable control that, when selected, causes the representation of the additional biological parameter to continue to be omitted from the user interface 302, while the representation 304 remains displayed in the user interface 302.

In addition to the notification 306, in some cases, the pacing device 300 may provide additional feedback to draw attention of the healthcare provider that other representations are available for display, such as visual feedback other than the notification 306 (e.g., a flashing LED light), audio feedback, haptic feedback, and so forth. Additionally, examples are considered in which the UI component 110 displays the additional biological parameters in the user interface 302 without first presenting a notification, as described above in relation to FIG. 1.

### EXAMPLE METHODS

Methods disclosed hereinafter are not claimed as such, but presented to exemplify how the invention can be used. These exemplary methods are useful for understanding the invention which is defined by the appended claims.

Various methods are described with reference to the example pacing device of FIG. 1 for convenience and ease of understanding. However, the methods described are not limited to being performed using the system of FIG. 1 and may be implemented using systems and devices other than those described herein.

The methods described herein represent sequences of operations that can be implemented in hardware, software, or a combination thereof. In the context of software, the blocks represent computer-executable instructions stored on one or more computer-readable storage media that, when executed by one or more processors, perform the recited operations. Generally, computer-executable instructions include routines, programs, objects, components, data structures, and the like that perform particular functions or implement particular abstract data types. The order in which the operations are described is not intended to be construed as a limitation, and any number of the described operations can be combined in any order and/or in parallel to implement the processes. In some examples, one or more operations of the method may be omitted entirely. Moreover, the methods described herein can be combined in whole or in part with each other or with other methods.

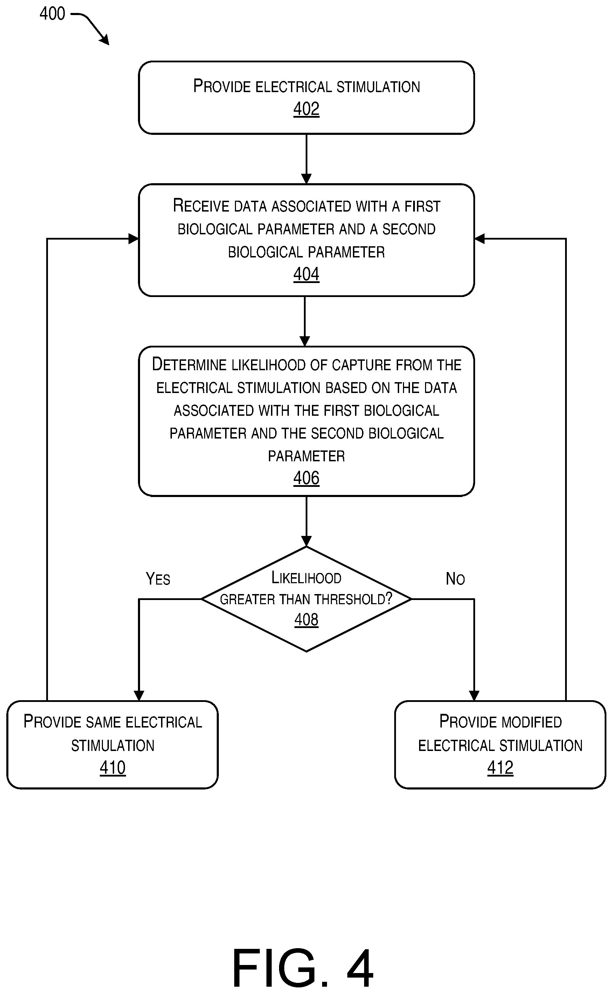

FIG. 4 is an example method 400 for providing electrical stimulation based on a likelihood of capture determined from multiple biological parameters, according to the techniques described herein.

At operation 402, the pacing device 100 provides an electrical stimulation to a patient. In examples, the electrical stimulation is one of multiple pulses provided during pacing, such as to attain capture of the heart of a patient experiencing bradycardia. For instance, the pacing component 104 causes a discharge circuit to deliver the electrical stimulation to electrodes attached to the patient via electrode leads connected to the pacing device 100. The pacing component 104 controls a current and/or a voltage of the electrical stimulation delivered to the electrodes, along with an interval at which the electrical stimulation is administered. In some cases, the electrical stimulation includes a train of stimuli, and/or a period of time (e.g., one heartbeat cycle length, one minute, etc.) during which electrical stimulation occurred.

At operation 404, the pacing device 100 receives data associated with a first biological parameter and data associated with a second biological parameter. The first biological parameter may correspond to ECG, SpO₂, NIBP, invasive blood pressure, ETCO₂, rSO₂, or ventilation, to name some examples. The second biological parameter may correspond to a different one of ECG, SpO₂, NIBP, invasive blood pressure, ETCO₂, rSO₂, or ventilation than the first biological parameter. The data associated with the first biological parameter and the data associated with the second biological parameter may be received at different times and/or intervals, such as for biological parameters that occur at different cycle rates (e.g., heartbeat and ventilation).

At operation 406, the pacing device 100 determines a likelihood of capture from the electrical stimulation based on the data associated with the first biological parameter and the data associated with the second biological parameter. In some cases, the capture component 106 employs a rules-based system to determine the likelihood that capture has been achieved. Such a rules-based system includes thresholds associated with different biological parameters (e.g., in a database or look-up table). The capture component 106 receives data associated with biological parameters before, during, and/or after pacing is administered to a patient, and compares values included in the data to the thresholds to determine a likelihood that capture has been achieved.

Alternatively or additionally, the capture component 106 may employ machine-learned model(s) 108 to determine the likelihood that capture has been achieved. The machine-learned models 108 also utilize thresholds associated with different biological parameters, but in some cases the parameters of the machine-learned models 108 are modified to improve accuracy of capture likelihood determinations. For instance, the capture component 106 receives data associated with biological parameters and inputs at least a portion of this data into the machine-learned models 108.

At operation 408, the pacing device 100 determines whether the likelihood of capture is greater than a threshold likelihood. The pacing component 104 compares the likelihood that capture has been achieved to at least one threshold likelihood of capture to determine whether the electrical current level of the electrical stimulation being delivered to the patient should change or remain the same for the upcoming electrical stimulation pulse. If the pacing device 100 determines that the likelihood is greater than or equal to the threshold likelihood (e.g., "Yes" at operation 408), the pacing device 100 provides a same electrical stimulation at operation 410. In this way, the electrical stimulation will remain at the same electrical current level if capture is achieved and the condition of the patient is improving (or not deteriorating). The method 400 then returns to the operation 404, at which the pacing device 100 receives additional data associated with the first biological parameter and the second biological parameter. The pacing device 100 continues to evaluate whether the electrical stimulations being administered to the patient are maintaining capture, by comparing a likelihood of capture to the threshold likelihood.

If the pacing device 100 determines that the likelihood is less than the threshold likelihood (e.g., "No" at operation 408), the pacing device 100 provides a modified electrical stimulation at operation 412. For example, if the likelihood of capture is less than the threshold, the pacing component 104 increases the electrical current level for an upcoming electrical stimulation (e.g., by 10 milliamps). The method 400 then returns to the operation 404, at which the pacing device 100 receives additional data associated with the first biological parameter and the second biological parameter. The pacing device 100 continues to evaluate whether the electrical stimulations being administered to the patient are achieving capture, by comparing a likelihood of capture to the threshold likelihood. For example, if capture is not achieved by increasing the electrical stimulation by 10 milliamps, the pacing component 104 increases the electrical current of the electrical stimulation again by another 10 milliamps.

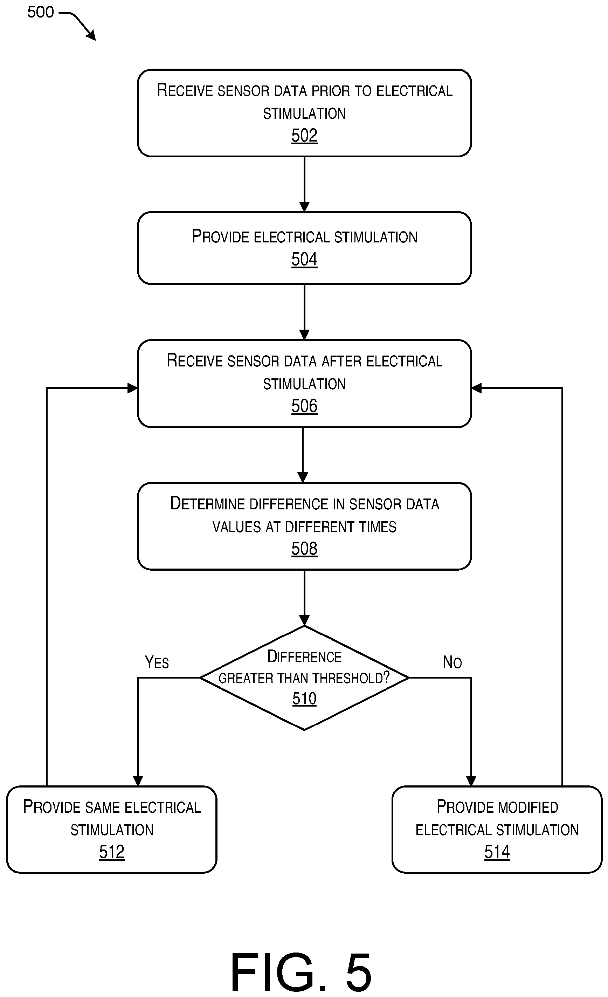

FIG. 5 is an example method 500 for providing electrical stimulation based on sensor data collected at different times, according to the techniques described herein. As described above, in some cases, the electrical stimulation includes a train of stimuli, and/or a period of time (e.g., one heartbeat cycle length, one minute, etc.) during which electrical stimulation occurred.

At operation 502, the pacing device 100 receives sensor data prior to an electrical stimulation. The sensor data is associated with at least one biological parameter, such as ECG, SpO₂, NIBP, invasive blood pressure, ETCO₂, rSO₂, and/or ventilation, to name some examples. In some examples, the sensor analysis component 102 receives the sensor data prior to administration of pacing, such as while electrodes are being placed on the patient, as vital signs are being collected, and so forth. The pacing device 100 stores the sensor data received prior to the electrical stimulation (also referred to as "historical sensor data") for later comparison with sensor data received during pacing. In some instances, the historical sensor data is from a past pacing setting, such as a previous electrical stimulation that was administered at a previous, different current amplitude. Alternatively or additionally, the historical sensor data is from data prior to achieving capture, and/or is from after capture has been achieved while monitoring to ensure that capture is maintained over time.

At operation 504, the pacing device 100 provides an electrical stimulation. The pacing component 104 may receive a user input to administer pacing to the patient, and subsequently deliver the electrical stimulation in accordance with pacing protocols. For instance, the pacing component 104 causes a discharge circuit to deliver the electrical stimulation to electrodes attached to the patient via electrode leads connected to the pacing device 100. The pacing component 104 controls a current and/or a voltage of the electrical stimulation delivered to the electrodes, along with an interval at which the electrical stimulation is administered.

At operation 506, the pacing device receives sensor data after the electrical stimulation. The sensor data received after the electrical stimulation is also associated with at least one biological parameter, such as ECG, SpO₂, NIBP, invasive blood pressure, ETCO₂, rSO₂, and/or ventilation, for instance.

At operation 508, the pacing device 100 determines a difference in sensor data values at different times, such as by comparing the sensor data received after the electrical stimulation to the sensor data received prior to the electrical stimulation. For example, the sensor analysis component 102 determines a difference between sensor data (e.g., an ECG pulse) generated after an electrical stimulation was administered and historical sensor data collected prior to administration of electrical stimulation during pacing.

At operation 510, the pacing device 100 determines whether the difference in sensor data values is greater than a threshold difference. If the difference between the sensor data after the electrical stimulation was administered and historical sensor data collected prior to administration of electrical stimulation during pacing is greater than or equal to a threshold difference (e.g., 50%, 20%, 10% difference, etc.), the capture component 106 determines that the electrical stimulation has likely caused capture. However, if the difference between the sensor data after the electrical stimulation was administered and historical sensor data collected prior to administration of electrical stimulation during pacing is less than the threshold difference, the capture component 106 determines that the electrical stimulation has likely failed to cause capture. Similar to the discussion above, the capture component 106 may input the difference into the machine-learned model 108, and receive a likelihood that the electrical stimulation caused capture from the machine-learned model based on the difference.

If the pacing device 100 determines that the difference is greater than or equal to the threshold difference (e.g., "Yes" at operation 510), the pacing device 100 provides a same electrical stimulation at operation 512. For example, the electrical stimulation will remain at the same electrical current level if capture is achieved and the condition of the patient is improving (or not deteriorating). The method 500 then returns to the operation 506, at which the pacing device 100 receives additional data associated with the biological parameter after the subsequent electrical stimulation. In some cases, the pacing component 104 determines differences between values associated with the biological parameter between pacing intervals in addition to or alternatively from determining the difference from the historical sensor data. Thus, the pacing component 104 tracks how capture is progressing over time with the administration of pacing.

If the pacing device 100 determines that the difference is less than the threshold difference (e.g., "No" at operation 510), the pacing device 100 provides a modified electrical stimulation at operation 514. The pacing component 104 then causes the electrical stimulation being administered to the patient to increase, as the electrical stimulation is likely insufficient to improve the magnitude of the patient's heartbeat. The method 500 then returns to the operation 506, at which the pacing device 100 receives additional data associated with the biological parameter after the subsequent modified electrical stimulation. In some examples, the pacing component 104 determines differences between values associated with the biological parameter between pacing intervals in addition to or alternatively from determining the difference from the historical sensor data. Thus, the pacing component 104 tracks whether the modification to the electrical current is causing capture to be achieved over time with the administration of pacing.

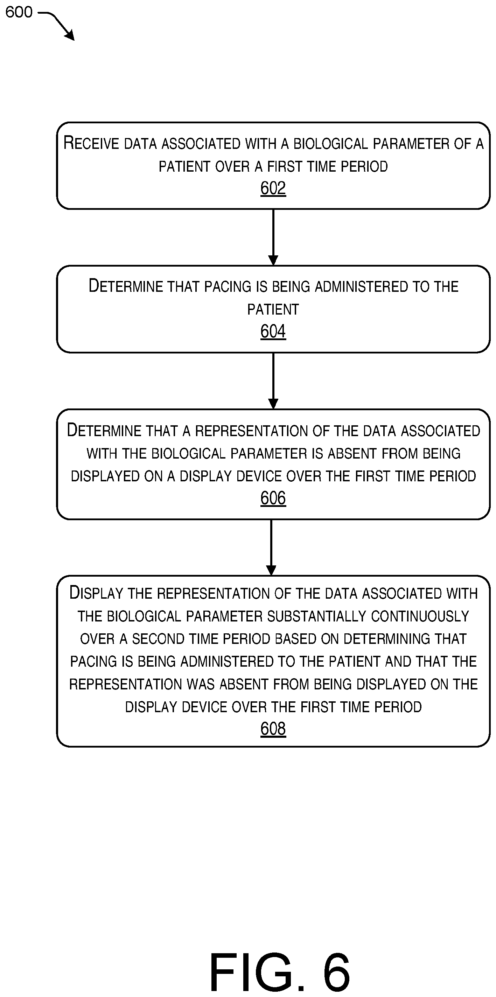

FIG. 6 is an example method 600 for displaying a representation of a biological parameter based on a determination that pacing is being administered, according to the techniques described herein.

At operation 602, the pacing device 100 receives data associated with a biological parameter of a patient over a first time period. Similar to the discussion above, the biological parameter may correspond to ECG, SpO₂, NIBP, invasive blood pressure, ETCO₂, rSO₂, or ventilation, to name some examples. In some cases, the pacing device 100 receives data associated with one or more additional biological parameters as well. Additional biological parameter(s) may correspond to a different one of ECG, SpO₂, NIBP, invasive blood pressure, ETCO₂, rSO₂, or ventilation than the first biological parameter. The first time period may correspond to a time period prior to administration of pacing, a time period after pacing began to be administered, or a combination of these.

At operation 604, the pacing device 100 determines that pacing is being administered. At operation 606, the pacing device determines that a representation of the data associated with the biological parameter is absent from being displayed on a display device over the first time period. For instance, the UI component 110 determines that a user interface being displayed on the display device includes information that is unrelated to the biological parameter, determines that information related to a different biological parameter is being displayed by the display device, or determines that the display is off, to name a few examples.

At operation 608, the pacing device 100 displays the representation of the data associated with the biological parameter substantially continuously over a second time period based on determining that pacing is being administered to the patient and that the representation was absent from being displayed on the display device over the first time period. For example, the second time period occurs after determining that pacing is being administered. In some examples, the UI component 110 determines that one or more different representations of biological parameters are currently being displayed than a predefined set of biological parameter representations that are helpful to identify capture. For instance, the predefined set of biological parameter representations may include ECG, SpO₂, and ETCO₂, although other examples are also considered (and are not limited to three representations). The UI component 110 may display a particular representation without input from a healthcare provider in response to the determination that pacing is being administered, to ensure that the healthcare provider is able to view representations of biological parameters that assist in pacing and capture determinations. In some examples, the UI component 110 displays a notification that a particular representation is absent from the user interface 112, then displays the particular representation in the user interface 112 based on selection of a selectable control to display the particular representation. Accordingly, healthcare providers are presented with one or more biological parameters that can assist with capture and pacing, without having to search through menus on the pacing device to select desired parameters to view.

### EXAMPLE EXTERNAL DEFIBRILLATOR

FIG. 7 illustrates an example of an external defibrillator 700 configured to perform various functions described herein. For example, the external defibrillator 700 is the pacing device 100 described above with reference to FIG. 1, as evidenced by inclusion of the sensor analysis component 102, the pacing component 104, and the UI component 110 in memory 714 of the external defibrillator 700. In some instances, the external defibrillator 700 detects arrhythmias such as bradycardia using the techniques described herein.

The external defibrillator 700 includes an electrocardiogram (ECG) port 702 connected to multiple ECG connectors 704. In some cases, the ECG connectors 704 are removeable from the ECG port 702. For instance, the ECG connectors 704 are plugged into the ECG port 702. The ECG connectors 704 are connected to ECG electrodes 706, respectively. In various implementations, the ECG electrodes 706 are disposed on different locations on an individual 708. A detection circuit 710 is configured to detect relative voltages between the ECG electrodes 706. These voltages are indicative of the electrical activity of the heart of the individual 708.

In various implementations, the ECG electrodes 706 are in contact with the different locations on the skin of the individual 708. In some examples, a first one of the ECG electrodes 706 is placed on the skin between the heart and right arm of the individual 708, a second one of the ECG electrodes 706 is placed on the skin between the heart and left arm of the individual 708, and a third one of the ECG electrodes 706 is placed on the skin between the heart and a leg (either the left leg or the right leg) of the individual 708. In these examples, the detection circuit 708 is configured to measure the relative voltages between the first, second, and third ECG electrodes 706. Respective pairings of the ECG electrodes 706 are referred to as "leads," and the voltages between the pairs of ECG electrodes 706 are known as "lead voltages." In some examples, more than three ECG electrodes 706 are included, such that 5-lead or 12-lead ECG signals are detected by the detection circuit 710.

The detection circuit 710 includes at least one analog circuit, at least one digital circuit, or a combination thereof. The detection circuit 710 receives the analog electrical signals from the ECG electrodes 706, via the ECG port 702 and the ECG leads 704. In some cases, the detection circuit 710 includes one or more analog filters configured to filter noise and/or artifact from the electrical signals. The detection circuit 710 includes an analog-to-digital (ADC) in various examples. The detection circuit 710 generates a digital signal indicative of the analog electrical signals from the ECG electrodes 706. This digital signal can be referred to as an "ECG signal" or an "ECG."

In some cases, the detection circuit 710 further detects an electrical impedance between at least one pair of the ECG electrodes 706. For example, the detection circuit 710 includes, or otherwise controls, a power source that applies a known voltage across a pair of the ECG electrodes 706 and detects a resultant current between the pair of the ECG electrodes 706. The impedance is generated based on the applied voltage and the resultant current. In various cases, the impedance corresponds to respiration of the individual 708, chest compressions performed on the individual 708, and other physiological states of the individual 708. In various examples, the detection circuit 710 includes one or more analog filters configured to filter noise and/or artifact from the resultant current. The detection circuit 710 generates a digital signal indicative of the impedance using an ADC. This digital signal can be referred to as an "impedance signal" or an "impedance."

The detection circuit 710 provides the ECG signal and/or the impedance signal one or more processors 712 in the external defibrillator 700. In some implementations, the processor(s) 712 includes a central processing unit (CPU), a graphics processing unit (GPU), both CPU and GPU, or other processing unit or component known in the art.

The processor(s) 712 is operably connected to memory 714. In various implementations, the memory 712 is volatile (such as random access memory (RAM)), non-volatile (such as read only memory (ROM), flash memory, etc.) or some combination of the two. The memory 714 stores instructions that, when executed by the processor(s) 712, causes the processor(s) 712 to perform various operations. In various examples, the memory 714 stores methods, threads, processes, applications, objects, modules, any other sort of executable instruction, or a combination thereof. In some cases, the memory 714 stores files, databases, or a combination thereof. In some examples, the memory 714 includes, but is not limited to, RAM, ROM, electrically erasable programmable readonly memory (EEPROM), flash memory, or any other memory technology. In some examples, the memory 714 includes one or more of CD-ROMs, digital versatile discs (DVDs), content-addressable memory (CAM), or other optical storage, magnetic cassettes, magnetic tape, magnetic disk storage or other magnetic storage devices, or any other medium which can be used to store the desired information and which can be accessed by the processor(s) 712 and/or the external defibrillator 700. In some cases, the memory 714 at least temporarily stores the ECG signal and/or the impedance signal.

In various examples, the memory 714 includes a detector 716, which causes the processor(s) 712 to determine, based on the ECG signal and/or the impedance signal, whether the individual 708 is exhibiting a particular heart rhythm. For instance, the processor(s) 712 determines whether the individual 708 is experiencing a shockable rhythm that is treatable by defibrillation. Examples of shockable rhythms include ventricular fibrillation (VF) and ventricular tachycardia (V-Tach). In some examples, the processor(s) 712 determines whether any of a variety of different rhythms (e.g., asystole, sinus rhythm, atrial fibrillation (AF), etc.) are present in the ECG signal.

The processor(s) 712 is operably connected to one or more input devices 718 and one or more output devices 720. Collectively, the input device(s) 718 and the output device(s) 720 function as an interface between a user and the defibrillator 700. The input device(s) 718 is configured to receive an input from a user and includes at least one of a keypad, a cursor control, a touch-sensitive display, a voice input device (e.g., a speaker), a haptic feedback device, or any combination thereof. The output device(s) 720 includes at least one of a display, a speaker, a haptic output device, a printer, or any combination thereof. In various examples, the processor(s) 712 causes a display among the input device(s) 718 to visually output a signal which may include a waveform of the ECG signal, and/or the impedance signal. In some implementations, the input device(s) 718 includes one or more touch sensors, the output device(s) 720 includes a display screen, and the touch sensor(s) are integrated with the display screen. Thus, in some cases, the external defibrillator 700 includes a touchscreen configured to receive user input signal(s) and visually output physiological parameters, such as the ECG signal and/or the impedance signal.

In some examples, the memory 714 includes an advisor 721, which, when executed by the processor(s) 712, causes the processor(s) 712 to generate advice and/or control the output device(s) 720 to output the advice to a user (e.g., a rescuer). In some examples, the processor(s) 712 provides, or causes the output device(s) 720 to provide, an instruction to perform CPR on the individual 708. In some cases, the processor(s) 712 evaluates, based on the ECG signal, the impedance signal, or other physiological parameters, CPR being performed on the individual 708 and causes the output device(s) 720 to provide feedback about the CPR in the instruction. According to some examples, the processor(s) 712, upon identifying that a shockable rhythm is present in the ECG signal, causes the output device(s) 720 to output an instruction and/or recommendation to administer a defibrillation shock to the individual 708.

The memory 714 also includes an initiator 723 which, when executed by the processor(s) 712, causes the processor(s) 712 to control other elements of the external defibrillator 700 in order to administer a defibrillation shock to the individual 708. In some examples, the processor(s) 712 executing the initiator 723 selectively causes the administration of the defibrillation shock based on determining that the individual 708 is exhibiting the shockable rhythm and/or based on an input from a user (received, e.g., by the input device(s) 718. In some cases, the processor(s) 712 causes the defibrillation shock to be output at a particular time, which is determined by the processor(s) 712 based on the ECG signal and/or the impedance signal. Alternatively or additionally, the initiator 723 causes the processor(s) 712 to administer electrical stimulations at intervals corresponding to pacing pulses, such as described above in relation to FIG. 1.

The processor(s) 712 is operably connected to a charging circuit 722 and a discharge circuit 724. In various implementations, the charging circuit 722 includes a power source 726, one or more charging switches 728, and one or more capacitors 730. The power source 726 includes, for instance, a battery. The processor(s) 712 initiates a defibrillation shock by causing the power source 726 to charge at least one capacitor among the capacitor(s) 730. For example, the processor(s) 712 activates at least one of the charging switch(es) 728 in the charging circuit 722 to complete a first circuit connecting the power source 726 and the capacitor to be charged. Then, the processor(s) 712 causes the discharge circuit 724 to discharge energy stored in the charged capacitor across a pair of defibrillation electrodes 730, which are in contact with the individual 708. For example, the processor(s) 712 deactivates the charging switch(es) 728 completing the first circuit between the capacitor(s) 730 and the power source 726, and activates one or more discharge switches 732 completing a second circuit connecting the charged capacitor 730 and at least a portion of the individual 708 disposed between defibrillation electrodes 734.

The energy is discharged from the defibrillation electrodes 734 in the form of a defibrillation shock. For example, the defibrillation electrodes 734 are connected to the skin of the individual 708 and located at positions on different sides of the heart of the individual 708, such that the defibrillation shock is applied across the heart of the individual 708. The defibrillation shock, in various examples, depolarizes a significant number of heart cells in a short amount of time. The defibrillation shock, for example, interrupts the propagation of the shockable rhythm (e.g., VF or V-Tach) through the heart. In some examples, the defibrillation shock is 200 J or greater with a duration of about 0.015 seconds. In some cases, the defibrillation shock has a multiphasic (e.g., biphasic) waveform. The discharge switch(es) 732 are controlled by the processor(s) 712, for example. In various implementations, the defibrillation electrodes 734 are connected to defibrillation connectors 736. The defibrillation connectors 736 are connected to a defibrillation port 738, in implementations. According to various examples, the defibrillation connectors 736 are removable from the defibrillation port 738. For example, the defibrillation connectors 736 are plugged into the defibrillation port 738.

In various implementations, the processor(s) 712 is operably connected to one or more transceivers 740 that transmit and/or receive data over one or more communication networks 742. For example, the transceiver(s) 740 includes a network interface card (NIC), a network adapter, a local area network (LAN) adapter, or a physical, virtual, or logical address to connect to the various external devices and/or systems. In various examples, the transceiver(s) 740 includes any sort of wireless transceivers capable of engaging in wireless communication (e.g., radio frequency (RF) communication). For example, the communication network(s) 742 includes one or more wireless networks that include a 3^{rd} Generation Partnership Project (3GPP) network, such as a Long Term Evolution (LTE) radio access network (RAN) (e.g., over one or more LE bands), a New Radio (NR) RAN (e.g., over one or more NR bands), or a combination thereof. In some cases, the transceiver(s) 740 includes other wireless modems, such as a modem for engaging in WI-FI^{®}, WIGIG^{®}, WIMAX^{®}, BLUETOOTH^{®}, or infrared communication over the communication network(s) 742.

The defibrillator 700 is configured to transmit and/or receive data (e.g., ECG data, impedance data, data indicative of one or more detected heart rhythms of the individual 708, data indicative of one or more defibrillation shocks administered to the individual 708, etc.) with one or more external devices 744 via the communication network(s) 742. The external devices 744 include, for instance, mobile devices (e.g., mobile phones, smart watches, etc.), Internet of Things (IoT) devices, medical devices, computers (e.g., laptop devices, servers, etc.), or any other type of computing device configured to communicate over the communication network(s) 742. In some examples, the external device(s) 744 is located remotely from the defibrillator 700, such as at a remote clinical environment (e.g., a hospital). According to various implementations, the processor(s) 712 causes the transceiver(s) 740 to transmit data to the external device(s) 744. In some cases, the transceiver(s) 740 receives data from the external device(s) 744 and the transceiver(s) 740 provide the received data to the processor(s) 712 for further analysis.

In various implementations, the external defibrillator 700 also includes a housing 746 that at least partially encloses other elements of the external defibrillator 700. For example, the housing 746 encloses the detection circuit 710, the processor(s) 712, the memory 714, the charging circuit 722, the transceiver(s) 740, or any combination thereof. In some cases, the input device(s) 718 and output device(s) 720 extend from an interior space at least partially surrounded by the housing 746 through a wall of the housing 746. In various examples, the housing 746 acts as a barrier to moisture, electrical interference, and/or dust, thereby protecting various components in the external defibrillator 700 from damage.

In some implementations, the external defibrillator 700 is an automated external defibrillator (AED) operated by an untrained user (e.g., a bystander, layperson, etc.) and can be operated in an automatic mode. In automatic mode, the processor(s) 712 automatically identifies a rhythm in the ECG signal, makes a decision whether to administer a defibrillation shock, charges the capacitor(s) 730, discharges the capacitor(s) 730, or any combination thereof. In some cases, the processor(s) 712 controls the output device(s) 720 to output (e.g., display) a simplified user interface to the untrained user. For example, the processor(s) 712 refrains from causing the output device(s) 720 to display a waveform of the ECG signal and/or the impedance signal to the untrained user, in order to simplify operation of the external defibrillator 700.

In some examples, the external defibrillator 700 is a monitor-defibrillator utilized by a trained user (e.g., a clinician, an emergency responder, etc.) and can be operated in a manual mode or the automatic mode. When the external defibrillator 700 operates in manual mode, the processor(s) 712 cause the output device(s) 720 to display a variety of information that may be relevant to the trained user, such as signals that include waveforms indicating the ECG data and/or impedance data, notifications about detected heart rhythms, and the like.

### CONCLUSION

As will be understood by one of ordinary skill in the art, each implementation disclosed herein can comprise, consist essentially of or consist of its particular stated element, step, or component. Thus, the terms "include" or "including" should be interpreted to recite: "comprise, consist of, or consist essentially of." The transition term "comprise" or "comprises" means has, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the implementation to the specified elements, steps, ingredients or components and to those that do not materially affect the implementation. As used herein, the term "based on" is equivalent to "based at least partly on," unless otherwise specified.

Unless otherwise indicated, all numbers expressing quantities, properties, conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing implementations (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate implementations of the disclosure and does not pose a limitation on the scope of the disclosure. No language in the specification should be construed as indicating any non-claimed element essential to the practice of implementations of the disclosure.

Groupings of alternative elements or implementations disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain implementations are described herein, including the best mode known to the inventors for carrying out implementations of the disclosure. Of course, variations on these described implementations will become apparent to those of ordinary skill in the art upon reading the foregoing description. The inventors expect skilled artisans to employ such variations as appropriate, and the inventors intend for implementations to be practiced otherwise than specifically described herein. Accordingly, the scope of the present invention is defined by the appended claims.

## Claims

1. An external pacing system, comprising:
electrodes (734) configured to deliver electrical stimulations to a patient via electrode leads;
a first biological sensor configured to sense a first biological parameter of the patient;
a second biological sensor configured to sense a second biological parameter of the patient, the second biological parameter being different than the first biological parameter;
a discharge circuit (724); and
a processor (712), wherein the processor is configured for:
controlling the discharge circuit to provide a first electrical stimulation at a current level to the electrodes at a first time;
receiving, from the first biological sensor at a second time after the first time, first data associated with the first biological parameter;
receiving, from the second biological sensor at the second time, second data associated with the second biological parameter, wherein the first biological parameter corresponds to ECG, SpO₂, NIBP, invasive blood pressure, ETCO₂, rSO₂, or ventilation, and wherein the second biological parameter corresponds to a different one of ECG, SpO₂, NIBP, invasive blood pressure, ETCO₂, rSO₂, or ventilation than the first biological parameter;
determining, based on the first data and the second data received at the second time, a likelihood that the first electrical stimulation has caused capture of a heart of the patient;
comparing the likelihood to a threshold likelihood; and
responsive to determining that the likelihood is greater than or equal to the threshold likelihood, controlling the discharge circuit to provide a second electrical stimulation at the current level by the discharge circuit to the electrodes.

2. The system of claim 1, wherein the first data associated with the first biological parameter comprises:
a T-wave or QRS complex identified in an electrocardiogram (ECG);
a plethysmography signal identified in an oxygen saturation (SpO₂) measurement;
a non-invasive blood pressure (NIBP) signal identified in an NIBP measurement;
a regional cerebral oxygen saturation (rSO₂) signal identified in an rSO₂ measurement;
an invasive blood pressure signal identified in an invasive blood pressure measurement;
an end-tidal CO₂ (ETCO₂) signal identified in an ETCO₂ measurement; or
a capnography signal identified in a respiratory measurement.

3. The system of claim 1 or 2, wherein the second data associated with the second biological parameter comprises a different one of the T-wave or the QRS complex, the plethysmography signal, the NIBP signal, the rSO₂ signal, the invasive blood pressure signal, the ETCO₂ signal, or the capnography signal than the first biological parameter.

4. The system of claim 1, 2 or 3, further comprising a user interface.

5. The system of claim 4, wherein the user interface is configured for:
displaying a first representation of the first biological parameter;
displaying a second representation of the second biological parameter with the first representation of the first biological parameter; and
displaying an indicator overlaying the first representation and the second representation at the first time at which the first electrical stimulation was provided.

6. The system of any of claims 1-5, wherein the processor is further configured for:
determining a first delay associated with receiving the first data from the first biological sensor; and
determining a second delay associated with receiving the second data from the second biological sensor,
wherein a location of the indicator relative to the first representation and the second representation is based on the first delay and the second delay.

7. The system of any preceding claim, wherein determining the likelihood comprises:
inputting the first data associated with the first biological parameter and the second data associated with the second biological parameter into a machine-learned model trained to identify heart capture; and
receiving, from the machine-learned model, the likelihood that the first electrical stimulation has caused the capture of the heart of the patient.

8. The system of claim 7, wherein the machine-learned model comprises a logistic regression model.

9. The system of any preceding claim, wherein the capture corresponds to electrical capture or mechanical capture of the heart of the patient.

10. The system of any preceding claim, wherein the processor is further configured for:
receiving, at a third time prior to the first time at which the electrical stimulation was provided, third data associated with the first biological parameter; and
determining a difference between the third data associated with the first biological parameter and first data associated with the biological parameter received at the second time,
wherein determining the likelihood is further based on the difference.

11. The system of claim 10, wherein determining the likelihood comprises:
inputting the first data associated with the first biological parameter and the difference into a machine-learned model trained to identify heart capture; and
receiving, from the machine-learned model, the likelihood that the first electrical stimulation has caused the capture of the heart of the patient.

12. The system of any preceding claim, wherein the first data associated with the first biological parameter comprises an indication of the electrical stimulation, the processor further being configured for:
determining a magnitude of a response of the patient to the electrical stimulation from the first data;
removing the magnitude of the response from the first data,
wherein determining the likelihood is further based on removing the magnitude of the response from the first data.

13. The system of claim 4, or any of claims 5-12 as far as dependent from claim 4, wherein the processor is configured for
responsive to determining that the likelihood is less than the threshold likelihood, displaying a suggestion in the user interface to provide a second electrical stimulation at a second current level different than the current level of the first electrical stimulation.

14. The system of claim 13, wherein the suggestion is a first suggestion and the first electrical stimulation is provided at a first timing interval, and wherein the user interface is further configured to display a second suggestion to provide the second electrical stimulation at a second timing interval, the second timing interval being different than the first timing interval.

15. Computer-readable media storing instructions that, when executed by is the processor of the system of any of claims 1-14, cause the processor to perform operations comprising:
providing a first electrical stimulation at a current level to an external surface of a patient by a discharge circuit via electrodes at a first time;
receiving, from a first biological sensor at a second time after the first time, first data associated with the first biological parameter;
receiving, from a second biological sensor at a second time, second data associated with a second biological parameter, wherein the first biological parameter corresponds to ECG, SpO₂, NIBP, invasive blood pressure, ETCO₂, rSO₂, or ventilation, and wherein the second biological parameter corresponds to a different one of ECG, SpO₂, NIBP, invasive blood pressure, ETCO₂, rSO₂, or ventilation than the first biological parameter;
determining, based on the first data and the second data received at the second time, a likelihood that the first electrical stimulation has caused capture of a heart of the patient;
comparing the likelihood to a threshold likelihood; and
responsive to determining that the likelihood is greater than or equal to the threshold likelihood, providing a second electrical stimulation at the current level by the discharge circuit via the electrodes to the patient.

## Patentansprüche

1. Externes Schrittmachersystem, umfassend:
Elektroden (734), dazu ausgestaltet, über Elektrodenleitungen einem Patienten elektrische Stimulationen zuzuführen;
einen ersten biologischen Sensor, dazu ausgestaltet, einen ersten biologischen Parameter des Patienten abzutasten;
einen zweiten biologischen Sensor, dazu ausgestaltet, einen zweiten biologischen Parameter des Patienten abzutasten, wobei sich der zweite biologische Parameter vom ersten biologischen Parameter unterscheidet;
eine Entladeschaltung (724); und
einen Prozessor (712), wobei der Prozessor ausgestaltet ist zum:
Steuern der Entladeschaltung, um den Elektroden zu einem ersten Zeitpunkt eine erste elektrische Stimulation mit einem aktuellen Niveau bereitzustellen;
Empfangen, vom ersten biologischen Sensor zu einem zweiten Zeitpunkt nach dem ersten Zeitpunkt, von ersten Daten, zugeordnet dem ersten biologischen Parameter;
Empfangen, vom zweiten biologischen Sensor zum zweiten Zeitpunkt, zweiter Daten, zugeordnet dem zweiten biologischen Parameter, wobei der erste biologische Parameter EKG, SpO₂, NIBP, invasivem Blutdruck, ETCO₂, rSO₂ oder Beatmung entspricht, und wobei der zweite biologische Parameter einem von EKG, SpO₂, NIBP, invasivem Blutdruck, ETCO₂, rSO₂ oder Beatmung entspricht, der vom ersten biologischen Parameter verschieden ist;
Bestimmen, basierend auf den ersten Daten und den zweiten Daten, empfangen zum zweiten Zeitpunkt, einer Wahrscheinlichkeit, dass die erste elektrische Stimulation eine Erfassung eines Herzens des Patienten verursacht hat;
Vergleichen der Wahrscheinlichkeit mit einer Schwellenwertwahrscheinlichkeit; und
in Reaktion auf das Feststellen, dass die Wahrscheinlichkeit größer als oder gleich der Schwellenwertwahrscheinlichkeit ist, Steuern der Entladungsschaltung, um den Elektroden eine zweite elektrische Stimulation auf dem aktuellen Niveau durch die Entladungsschaltung bereitzustellen.

2. System nach Anspruch 1, wobei die ersten Daten, zugeordnet dem ersten biologischen Parameter, umfassen:
einen T-Wellen- oder QRS-Komplex, identifiziert in einem Elektrokardiogramm (EKG);
ein Plethysmographiesignal, identifiziert in einer Messung der Sauerstoffsättigung (SPO₂);
ein Signal des nichtinvasiven Blutdrucks (NIBP), identifiziert in einer NIBP-Messung;
ein Signal der regional-zerebralen Sauerstoffsättigung (rSO₂), identifiziert in einer rSO₂ -Messung;
ein Signal des invasiven Blutdrucks, identifiziert in einer invasiven Blutdruckmessung;
ein Signal des endtidalen CO₂ (ETCO₂), identifiziert in einer ETCO₂ - Messung; oder
ein Kapnographiesignal, identifiziert in einer respiratorischen Messung.

3. System nach Anspruch 1 oder 2, wobei die zweiten Daten, zugeordnet dem zweiten biologischen Parameter, einen anderen von dem T-Wellen- oder QRS-Komplex, dem Plethysmographiesignal, dem NIBP-Signal, dem rSO2-Signal, dem invasiven Blutdrucksignal, dem ETCO₂-Signal oder dem Kapnographiesignal umfassen als der erste biologische Parameter.

4. System nach Anspruch 1, 2 oder 3, ferner umfassend eine Benutzeroberfläche.

5. System nach Anspruch 4, wobei die Benutzeroberfläche ausgestaltet ist für:
Anzeigen einer ersten Darstellung des ersten biologischen Parameters;
Anzeigen einer zweiten Darstellung des zweiten biologischen Parameters mit der ersten Darstellung des ersten biologischen Parameters; und
Anzeigen eines Indikators, überlappend die erste Darstellung und die zweite Darstellung zum ersten Zeitpunkt, zu dem die erste elektrische Stimulation bereitgestellt wurde.

6. System nach einem der Ansprüche 1 bis 5, wobei der Prozessor ferner konfiguriert ist für:
Bestimmen einer ersten Verzögerung im Zusammenhang mit dem Empfangen der ersten Daten vom ersten biologischen Sensor; und
Bestimmen einer zweiten Verzögerung im Zusammenhang mit dem Empfangen der zweiten Daten vom zweiten biologischen Sensor,
wobei eine Position des Indikators relativ zur ersten Darstellung und zur zweiten Darstellung auf der ersten Verzögerung und der zweiten Verzögerung basiert.

7. System nach einem der vorhergehenden Ansprüche, wobei die Bestimmung der Wahrscheinlichkeit umfasst:
Eingeben der ersten Daten, zugeordnet dem ersten biologischen Parameter, und der zweiten Daten, zugeordnet dem zweiten biologischen Parameter, in ein maschinell gelerntes Modell, trainiert zum Identifizieren von Herzerfassung; und
Erhalten, aus dem maschinell gelernten Modell, der Wahrscheinlichkeit, dass die erste elektrische Stimulation das Erfassen des Herzens des Patienten verursacht hat.

8. System nach Anspruch 7, wobei das maschinell gelernte Modell ein logistisches Regressionsmodell umfasst.

9. System nach einem der vorhergehenden Ansprüche, wobei die Erfassung der elektrischen Erfassung oder der mechanischen Erfassung des Herzens des Patienten entspricht.

10. System nach einem der vorhergehenden Ansprüche, wobei der Prozessor ferner ausgestaltet ist für:
Empfangen, zu einem dritten Zeitpunkt vor dem ersten Zeitpunkt, zu dem die elektrische Stimulation bereitgestellt wurde, von dritten Daten, zugeordnet dem ersten biologischen Parameter; und
Bestimmen einer Differenz zwischen den dritten Daten, zugeordnet dem ersten biologischen Parameter, und ersten Daten, zugeordnet dem zum zweiten Zeitpunkt empfangenen biologischen Parameter,
wobei das Bestimmen der Wahrscheinlichkeit ferner auf der Differenz beruht.

11. Verfahren nach Anspruch 10, wobei das Bestimmen der Wahrscheinlichkeit umfasst:
Eingeben der dem ersten biologischen Parameter zugeordneten ersten Daten und der Differenz in ein maschinell gelerntes Modell, trainiert zur Identifizierung der Herzerfassung; und
Erhalten, aus dem maschinell gelernten Modell, der Wahrscheinlichkeit, dass die erste elektrische Stimulation das Erfassen des Herzens des Patienten verursacht hat.

12. System nach einem der vorhergehenden Ansprüche, wobei die dem ersten biologischen Parameter zugeordneten ersten Daten eine Indikation der elektrischen Stimulation umfassen, wobei der Prozessor ferner konfiguriert ist für:
Bestimmen einer Größenordnung einer Reaktion des Patienten auf die elektrische Stimulation aus den ersten Daten;
Entfernen der Größenordnung der Reaktion aus den ersten Daten,
wobei das Bestimmen der Wahrscheinlichkeit ferner auf dem Entfernen der Größenordnung der Reaktion aus den ersten Daten basiert.

13. System nach Anspruch 4 oder einem der Ansprüche 5 bis 12, sofern abhängig von Anspruch 4, wobei der Prozessor ausgestaltet ist für
das Anzeigen, in Reaktion auf das Feststellen, dass die Wahrscheinlichkeit kleiner als die Schwellenwertwahrscheinlichkeit ist, eines Vorschlags in der Benutzernutzeroberfläche, eine zweite elektrische Stimulation auf einem zweiten aktuellen Niveau abzugeben, das sich vom aktuellen Niveau der ersten elektrischen Stimulation unterscheidet.

14. System nach Anspruch 13, wobei der Vorschlag ein erster Vorschlag ist und die erste elektrische Stimulation in einem ersten Zeitintervall bereitgestellt ist, und wobei die Benutzeroberfläche ferner dazu ausgestaltet ist, einen zweiten Vorschlag zur Bereitstellung der zweiten elektrischen Stimulation in einem zweiten Zeitintervall anzuzeigen, wobei sich das zweite Zeitintervall vom ersten Zeitintervall unterscheidet.

15. Computerlesbare Medien zum Speichern von Anweisungen, die, wenn sie vom Prozessor des Systems nach einem der Ansprüche 1 bis 14 ausgeführt werden, den Prozessor dazu veranlassen, Vorgänge auszuführen, umfassend:
Bereitstellen einer ersten elektrischen Stimulation auf einem aktuellen Niveau an eine Außenfläche eines Patienten durch eine Entladungsschaltung über Elektroden zu einem ersten Zeitpunkt;
Empfangen, von einem ersten biologischen Sensor zu einem zweiten Zeitpunkt nach dem ersten Zeitpunkt, von ersten Daten, zugeordnet dem ersten biologischen Parameter;
Empfangen, von einem zweiten biologischen Sensor zu einem zweiten Zeitpunkt, zweiter Daten, zugeordnet einem zweiten biologischen Parameter, wobei der erste biologische Parameter EKG, SpO₂, NIBP, invasivem Blutdruck, ETCO₂, rSO₂ oder Beatmung entspricht, und wobei der zweite biologische Parameter einem von EKG-Parameter, SpO₂, NIBP, invasivem Blutdruck, ETCO₂, rSO₂ oder Beatmung, vom ersten biologischen Parameter verschieden, entspricht;
Bestimmen, basierend auf den ersten Daten und den zweiten Daten, empfangen zum zweiten Zeitpunkt, einer Wahrscheinlichkeit, dass die erste elektrische Stimulation eine Erfassung eines Herzens des Patienten verursacht hat;
Vergleichen der Wahrscheinlichkeit mit einer Schwellenwertwahrscheinlichkeit; und
in Reaktion auf das Feststellen, dass die Wahrscheinlichkeit größer/gleich der Schwellenwertwahrscheinlichkeit ist, Bereitstellen einer zweiten elektrischen Stimulation auf dem aktuellen Niveau über die Elektroden durch die Entladungsschaltung an den Patienten.

## Revendications

1. Système de stimulation externe, comprenant :
des électrodes (734) configurées pour délivrer des stimulations électriques à un patient via des fils conducteurs d'électrode ;
un premier capteur biologique configuré pour détecter un premier paramètre biologique du patient ;
un deuxième capteur biologique configuré pour détecter un deuxième paramètre biologique du patient, le deuxième paramètre biologique étant différent du premier paramètre biologique ;
un circuit de décharge (724) ; et
un processeur (712), dans lequel le processeur est configuré pour :
commander le circuit de décharge pour fournir une première stimulation électrique à un niveau de courant aux électrodes à un premier moment ;
recevoir, du premier capteur biologique à un deuxième moment après le premier moment, des premières données associées au premier paramètre biologique ;
recevoir, du deuxième capteur biologique au deuxième moment, des deuxièmes données associées au deuxième paramètre biologique, dans lequel le premier paramètre biologique correspond à ECG, SpO₂, PNI, pression artérielle invasive, ETCO₂, rSO₂, ou ventilation, et dans lequel le deuxième paramètre biologique correspond à un de ECG, SpO₂, PNI, pression artérielle invasive, ETCO₂, rSO₂, ou ventilation différent du premier paramètre biologique ;
déterminer, sur la base des premières données et des deuxièmes données reçues au deuxième moment, d'une probabilité que la première stimulation électrique ait provoqué la capture d'un cœur du patient ;
comparer la probabilité à un seuil de probabilité ; et
en réponse à la détermination que la probabilité est supérieure ou égale au seuil de probabilité, commander le circuit de décharge pour fournir une deuxième stimulation électrique au niveau de courant par le circuit de décharge aux électrodes.

2. Système selon la revendication 1, dans lequel les premières données associées au premier paramètre biologique comprennent :
une onde T ou un complexe QRS identifié(e) dans un électrocardiogramme (ECG) ;
un signal de pléthysmographie identifié dans une mesure de saturation en oxygène (SpO₂) ;
un signal de pression artérielle non invasive (PNI) identifié dans une mesure de PNI ;
un signal de saturation en oxygène cérébral (rSO₂) régional identifié dans une mesure de rSO₂ ;
un signal de pression artérielle invasive identifié dans une mesure de pression artérielle invasive ;
un signal de CO₂ en fin d'expiration (ETCO₂) identifié dans une mesure ETCO₂ ; ou
un signal de capnographie identifié dans une mesure respiratoire.

3. Système selon la revendication 1 ou 2, dans lequel les deuxièmes données associées au deuxième paramètre biologique comprennent un de l'onde T ou du complexe QRS, du signal de pléthysmographie, du signal PNI, du signal rSO₂, du signal de pression sanguine invasive, du signal ETCO₂ ou du signal de capnographie différent du premier paramètre biologique.

4. Système selon la revendication 1, 2 ou 3, comprenant en outre une interface utilisateur.

5. Système selon la revendication 4, dans lequel l'interface utilisateur est configurée pour :
afficher une première représentation du premier paramètre biologique ;
afficher une deuxième représentation du deuxième paramètre biologique avec la première représentation du premier paramètre biologique ; et
afficher un indicateur superposant la première représentation et la deuxième représentation au premier moment auquel la première stimulation électrique a été fournie.

6. Système selon l'une quelconque des revendications 1-5, dans lequel le processeur est en outre configuré pour :
déterminer un premier délai associé à la réception des premières données du premier capteur biologique ; et
déterminer un deuxième délai associé à la réception des deuxièmes données du deuxième capteur biologique,
dans lequel un emplacement de l'indicateur par rapport à la première représentation et à la deuxième représentation est basé sur le premier délai et le deuxième délai.

7. Système selon une quelconque revendication précédente, dans lequel la détermination de la probabilité comprend :
la saisie des premières données associées au premier paramètre biologique et des deuxièmes données associées au deuxième paramètre biologique dans un modèle d'apprentissage automatique entraîné pour identifier la capture cardiaque ; et
la réception, du modèle d'apprentissage automatique, de la probabilité que la première stimulation électrique ait provoqué la capture du cœur du patient.

8. Système selon la revendication 7, dans lequel le modèle d'apprentissage automatique comprend un modèle de régression logistique.

9. Système selon une quelconque revendication précédente, dans lequel la capture correspond à la capture électrique ou capture mécanique du cœur du patient.

10. Système selon une quelconque revendication précédente, dans lequel le processeur est en outre configuré pour :
recevoir, à un troisième moment avant le premier moment auquel la stimulation électrique a été fournie, des troisièmes données associées au premier paramètre biologique ; et
déterminer une différence entre les troisièmes données associées au premier paramètre biologique et les premières données associées au paramètre biologique reçues au deuxième moment,
dans lequel la détermination de la probabilité est en outre basée sur la différence.

11. Système selon la revendication 10, dans lequel la détermination de la probabilité comprend :
la saisie des premières données associées au premier paramètre biologique et à la différence dans un modèle d'apprentissage automatique entraîné pour identifier la capture cardiaque ; et
la réception, du modèle d'apprentissage automatique, de la probabilité que la première stimulation électrique ait provoqué la capture du cœur du patient.

12. Système selon une quelconque revendication précédente, dans lequel les premières données associées au premier paramètre biologique comprennent une indication de la stimulation électrique, le processeur étant en outre configuré pour :
déterminer une amplitude d'une réponse du patient à la stimulation électrique à partir des premières données ;
éliminer l'amplitude de la réponse des premières données,
dans lequel la détermination de la probabilité est en outre basée sur l'élimination de l'amplitude de la réponse des premières données.

13. Système selon la revendication 4, ou l'une quelconque des revendications 5-12 dans la mesure où elle dépend de la revendication 4, dans lequel le processeur est configuré pour
en réponse à la détermination que la probabilité est inférieure au seuil de probabilité, afficher une suggestion dans l'interface utilisateur pour fournir une deuxième stimulation électrique à un deuxième niveau de courant différent du niveau de courant de la première stimulation électrique.

14. Système selon la revendication 13, dans lequel la suggestion est une première suggestion et la première stimulation électrique est fournie à un premier intervalle de synchronisation, et dans lequel l'interface utilisateur est en outre configurée pour afficher une deuxième suggestion pour fournir la deuxième stimulation électrique à un deuxième intervalle de synchronisation, le deuxième intervalle de synchronisation étant différent du premier intervalle de synchronisation.

15. Support lisible par ordinateur stockant des instructions qui, lorsqu'elles sont exécutées par le processeur du système selon l'une quelconque des revendications 1-14, amènent le processeur à effectuer des opérations comprenant :
la fourniture d'une première stimulation électrique à un niveau de courant à une surface externe d'un patient par un circuit de décharge via des électrodes à un premier moment ;
la réception, d'un premier capteur biologique à un deuxième moment après le premier moment, de premières données associées au premier paramètre biologique ;
la réception, d'un deuxième capteur biologique à un deuxième moment, de deuxièmes données associées à un deuxième paramètre biologique, dans lequel le premier paramètre biologique correspond à ECG, SpO₂, PNI, pression artérielle invasive, ETCO₂, rSO₂, ou ventilation, et dans lequel le deuxième paramètre biologique correspond à un de ECG, SpO₂, PNI, pression artérielle invasive, ETCO₂, rSO₂, ou ventilation différent du premier paramètre biologique ;
la détermination, sur la base des premières données et des deuxièmes données reçues au deuxième moment, d'une probabilité que la première stimulation électrique ait provoqué la capture d'un cœur du patient ;
la comparaison de la probabilité à un seuil de probabilité ; et
en réponse à la détermination que la probabilité est supérieure ou égale au seuil de probabilité, la fourniture d'une deuxième stimulation électrique au niveau de courant par le circuit de décharge via les électrodes au patient.
